(19) Europäisches Patentamt / European Patent Office / Office européen des brevets

(11) **EP 3 675 001 B1**

(12) **EUROPEAN PATENT SPECIFICATION**

(45) Date of publication and mention of the grant of the patent:
**16.04.2025 Bulletin 2025/16**

(21) Application number: **18382999.3**

(22) Date of filing: **27.12.2018**

(51) International Patent Classification (IPC):
**G06Q 10/00** (2023.01)

(52) Cooperative Patent Classification (CPC):
**G06Q 10/00**

(54) **A COMPUTER IMPLEMENTED METHOD, A SYSTEM AND COMPUTER PROGRAM FOR DETERMINING OPTIMAL BEHAVIOR PATH FOR A USER**

COMPUTERIMPLEMENTIERTES VERFAHREN, SYSTEM UND COMPUTERPROGRAMM ZUR BESTIMMUNG DES OPTIMALEN VERHALTENSPFADES FÜR EINEN BENUTZER

PROCÉDÉ MIS EN ŒUVRE PAR ORDINATEUR, SYSTÈME ET PROGRAMME INFORMATIQUE POUR DÉTERMINER LE CHEMIN DE COMPORTEMENT OPTIMAL POUR UN UTILISATEUR

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**01.07.2020 Bulletin 2020/27**

(73) Proprietor: **Koa Health Digital Solutions S.L.U.**
**08018 Barcelona (ES)**

(72) Inventors:
• **Khwaja, Mohammed**
  **28013 Madrid (ES)**
• **Matic, Aleksandar**
  **28013 Madrid (ES)**

(74) Representative: **Carlos Hernando, Borja**
**Garrigues IP, S.L.P.**
**Hermosilla, 3**
**28001 Madrid (ES)**

(56) References cited:
CN-A- 107 702 706    US-A1- 2007 233 631
US-A1- 2014 335 490    US-A1- 2017 049 963

**Description**

## TECHNICAL FIELD OF THE INVENTION

Present invention generally relates to computing methods and systems. In particular, the invention relates to a method, system and computer programs, for determining optimal behavior path for a user, such that personalized recommendations about lifestyle modifications (i.e. change the activities the user normally does and/or their behavior) can be then given to the user to improve the user's wellbeing. The present application has received funding from the European Union's Horizon 2020 research and innovation programme under the Marie Skłodowska-Curie grant agreement No 722561.

## BACKGROUND OF THE INVENTION

US 20170132395-A1 provides a connected digital therapeutics navigation guidance system for lifestyle modification and disease prevention. The system includes one or more wearable user devices to detect user data relating to one or more health or fitness parameters, receive user input data relating to the one or more health or fitness parameters through the user interface and a request for a health or fitness routine from a server, which provides the wearable user device with a routine that can be performed by the user of the wearable user device.

US 2012059785-A1 relates to a content recommendation method and system based on psychological factors from a user profile, which comprises: (a) a website for downloads that is accessible for the user, where said site comprises means configured for the detection of his consumption profile; (b) a psychographics driver configured to calculate his profile in the five super traits of the Big Five model, said profile being stored in a first database, said data being accessible for the current query and subsequent queries; and (c) means configured to cross-match the psychological profile stored in the first database and the data contained in a second content database; all such that access preferably to those contents which best adapt to the calculated psychological profile is granted to the user.

US 7457768-B2 describes a new recommendation technique that can be seen as a hybrid between memory-based and model-based collaborative filtering techniques. Using personality diagnosis, all data may be maintained throughout the processes, new data can be added incrementally, and predictions have meaningful probabilistic semantics. Each entity's (e.g. user's) reported attributes (e.g., item ratings or preferences) may be interpreted as a manifestation of their underlying personality type. Personality type may be encoded simply as a vector of the entity's (e.g. user's) "true" values (e.g. ratings) for attributes (e.g. items) in the database. It may be assumed that entities (e.g. users) report values (e.g. ratings) with a distributed (e.g. Gaussian) error. Given an active entity's (e.g., user's) known attribute values (e.g. item ratings), the probability that they have the same personality type as every other entity (e.g. user) may be determined. Then, the probability that they will have a given value (e.g. rating) for a valueless (e.g. unrated) attribute (e.g. item) may then be determined based on the entity's (e.g. user's) personality type. The probabilistic determinations may be used to determine expected value of information. Such an expected value of information could be used in at least two ways. First, an interactive recommender could use expected value of information to favorably order queries for attribute values (e.g. item ratings), thereby mollifying what could otherwise be a tedious and frustrating process. Second, expected value of information could be used to determine which entries of a database to prune or ignore-that is, which entries, which if removed, would have a minimal effect of the accuracy of recommendations.

CN 103309976-B discloses a method for improving the social recommendation efficiency based on user personality. The method comprises the steps as follows: determining k commodities to be recommended to a target user; for each commodity *m* to be recommended, calculating the comprehensive recommendation scores of the good friends of the target user based on personalities and commodity preferences respectively, and selecting the friend with the highest score as a corresponding recommender of the commodity m; and notifying each recommender to recommend the corresponding commodity to the target user. According to the method, the proper recommender is selected according to the user personality features and the commodity preferences, so that the recommendation efficiency is improved.

Document US 2017/049663 A1 discloses providing medical devices and patient management systems and parameter modeling methods.

Document US 2014/335490 A1 relates to a behavior modification system, including a network of components that interact to collect various data and provide user feedback.

Document US 2007/233631 A1 refers to a behavior predicting apparatus including an input unit including inputting sole behaviors of a human and simultaneous occurrence probability of behaviors.

Document CN 107 702 706 A discloses a path determination method, a device, a storage medium and a mobile terminal.

The known collaborative filtering systems based on personality do not dynamically calculate personality from behaviors and compare this to static personality traits to form similarities. New methods and systems for determining optimal behavior path for a user are therefore needed enabling incorporation of known and unknown psychological constructs and personal characteristics.

## DESCRIPTION OF THE INVENTION

To that end, embodiments of the present invention provide, according to a first aspect, a computer implemented method for determining optimal behavior path for a user. The method is implemented by a computing system including at least one memory and one or more processors. The method comprises: receiving first data regarding different personal characteristics of a user, providing a first vector of personal characteristics of said user based on said received first data; receiving second data regarding behavior and activity characteristics of the user, providing a second vector of behavior path characteristics of the user based on said received second data; and receiving third data regarding one or more subjective wellbeing measures, also termed SWB, of the user, providing a third vector of wellbeing measures of the user based on said received third data.

Then, the computing system calculates exhibited personal characteristics of the user using the first and second vectors of the user, and calculates a miss-alignment parameter between the calculated exhibited personal characteristics and the first vector of the user.

The computer system also creates a reference group for the user, said reference group comprising a plurality of users having a higher third vector and lower miss-alignment parameter than the user, and implements a similarity measure between the user and the reference group to identify which of said plurality of users within the reference group has more characteristics in common with the user.

Finally, the computing system determines a user's behavior path vector from the most similar users of said reference group. The determined user's behavior path vector can be used to recommend behavior path modifications for the user.

In an embodiment, the exhibited personal characteristics of the user are calculated by calculating a user's behavior path distribution matrix LDM by concatenating the second vector with second vectors of other users stored in a database; calculating a correlation matrix C by correlating the first vector with the second vector; calculating a weight matrix by implementing the following equation $W = LDM \times C$, where $\times$ is the matrix multiplication operator; and calculating the exhibited personal characteristics by implementing the following equation: $\overrightarrow{PC}^{(j)}_{exhibited} = \overrightarrow{PC}_{med} + \overrightarrow{PC}_{med} \cdot \overrightarrow{w}^{(j)} = \overrightarrow{PC}_{med}(1 + \overrightarrow{w}^{(j)})$, where $\overrightarrow{PC}_{med}$ is the median of the first vector and $(\cdot)$ is the Hadamard product of the weights of the user with said median of the first vector.

In an embodiment, the similarity measure is calculated by comparing the first vector of the user with a first vector of each one of the plurality of users within the reference group using a cosine similarity measure. In this case, the first vector of each one of the plurality of users being calculated using personal characteristics of each user.

In another embodiment, the similarity measure is calculated by comparing the first vector of the user with calculated exhibited personal characteristics of each one of the plurality of users within the reference group using a cosine similarity measure. In this case, the exhibited personal characteristics of each one of the plurality of users being calculated using personal characteristics and behavior and activity characteristics of each user.

In a particular embodiment, the first data is received from a server providing results of a personality survey implemented by the user. Optionally, the first data can be further inferred through knowledge of variables of the user including people from a same neighborhood or community and/or by using a clustering or machine learning algorithm on said variables.

In an embodiment, the second data is received as a vector quantifying a proportion of the different daily activities of the user where each activity is assigned with a specific value or with a specific percentage.

In a particular embodiment, the second data is received from a server providing a self-report of the user and/or from a passive sensor, mobile phone, wearable device or activity recognition system of the user. The third data can be received from a sensor monitoring activities or physiological signals of the user including body temperature, heart rate parameters, voice parameters and/or facial expressions.

Other embodiments of the invention provide, according to other aspects, a system and computer program products including instructions embodied in a non-transitory computer readable medium that, when executed by a processor, cause the processor to determine optimal behavior path for a user.

Present invention relies on the psychological principle that for an individual living life in line with "who he/she is" in terms of personal characteristics (such as personality, demographics, psychological profiles, biological characteristics, etc.) leads to an improved SWB. There are individuals whose lifestyle (i.e. behavior and activities) is aligned with their personal characteristics and who have a high SWB and there are individuals whose lifestyle is not aligned with their personal characteristics and who have low SWB. Assessing the "alignment" between behavior and activity characteristics and personal characteristics is performed using the concept of "exhibited personal characteristics" in which modelling personal characteristics is proposed based on the observed behavior path and comparing that exhibited personal characteristic with the actual one (known from another source, such as a survey, questionnaire, clustering method, belonging to a specific demographics, etc.) aiming to reduce the gap between the two by modifying behavior path of the user.

In other words, present invention dynamically models personal characteristics of a user/individual based on observed behaviors and activities of the user. The method infers if the user's lifestyle is not aligned with his/her personal characteristics and can recommend changes to live closer to an optimal lifestyle. The optimal behavior path is inferred through the analysis of characteristics of users with similar personal characteristics to the user who are positive role models with respect to the quality of life measured as a subjective wellbeing. Thus, present invention can be used as a recommendation system for modifying time distribution of daily activities in one's life.

## BRIEF DESCRIPTION OF THE DRAWINGS

Fig. 1 schematically illustrates the different elements and/or tools that can be used for capturing the personal characteristics and the behaviors and activities of the user.

Figs. 2 and 3 schematically illustrate different embodiments for calculating the similarity measures between the user and the users within the reference group.

## DETAILED DESCRIPTION OF THE INVENTION AND OF PREFERRED EMBODIMENTS

Present invention provides a method, and corresponding system, for determining optimal behavior path for a user. In particular, the method relies on four main actions:

- Capturing or receiving actual personal characteristics of the user.
- Capturing or receiving user's behaviors and activities (actual lifestyle) and SWB indices/characteristics.
- Quantifying exhibited personal characteristics and computing a miss-alignment value/parameter.
- Finding a reference group and preferably, establishing the recommendation.

Capturing personal characteristics

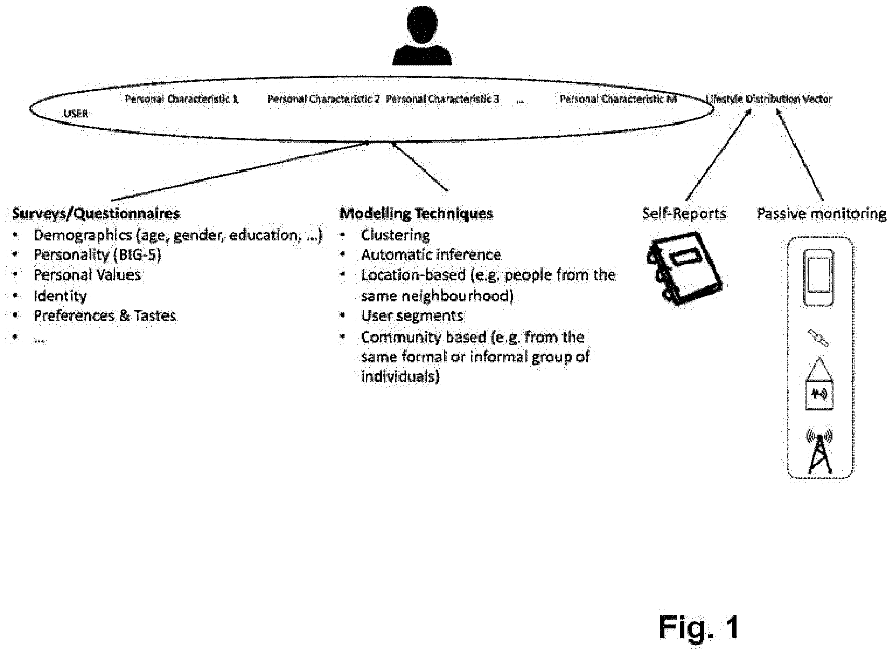

Personal characteristics (i.e. the first data as termed in the claims) can be self-reported through surveys or questionnaires using validated or non-validated inventories (see Fig. 1). For instance, personality traits can be acquired through asking the user to fill out the validated BIG-5 personality questionnaire (which results in five components of personality i.e. five different personal characteristics - Extroversion, Agreeableness, Conscientiousness, (Emotional) Stability and Intellect). Personal characteristics can be also inferred indirectly through knowledge of other variables or by using these variables with clustering or machine learning techniques based on such variables. The invention can rely on one or more personal characteristics acquired in one or more ways.

Capturing user's behaviors and activities and SWB

Lifestyle represents a description of users' typical activities and behaviors in life. This can be a vector (second vector as termed in the claims) that quantifies proportion of different activities like, for example, sleeping, driving, reading, watching TV.... -> 23, 12, 31, 52... where each activity can be assigned with a specific number of points (openly defined for a specific implementation of present invention), or in percentages (26%, 4%, 5%, 1%, ...) that defines proportion of life in an observed period of time that the user spends on specific categories of activities.

Observing user's behaviors and activities can be conducted using typical self-reporting strategies, such as diaries, (daily) reconstruction methods, ecological momentary assessments, etc., aggregated over a specific period of time (such as a few weeks, months, years) to obtain the user's behavior path vector (or lifestyle distribution vector (LDV) as referred in the figures).

Activities and behaviors can be also captured using passive sensors, mobile phones, wearables, environmental sensors and/or activity recognition systems.

LDV can combine activities acquired in a passive way or through self-report methods.

On the other hand, SWB can include one or the combination of the following wellbeing measures, namely: evaluative wellbeing (life satisfaction), hedonic wellbeing (sense of pleasure) and eudemonic wellbeing (sense of purpose) scores, and can be acquired once or multiple times in self-reported ways. SWB can be also modelled through data acquired from passive monitoring of activities or physiological signals (this includes for example body temperature, heart rate parameters such as heart rate variability, non-verbal voice analysis, video analysis of facial expressions, etc.).

Thus, for each user $j$, the above produces three vectors:

1. First vector of $m$ Personal Characteristics: $\overrightarrow{PC}^{(j)} = <PC_1^{(j)}, PC_2^{(j)}, PC_3^{(j)}, ... PC_m^{(j)}>$

2. Second vector of $k$ behavioral categories: $\overrightarrow{LDV}^{(j)} = <BC_1^{(j)}, BC_2^{(j)}, BC_3^{(j)}, ... BC_k^{(j)}>$

3. Third vector of $p$ subjective wellbeing measures:

$$\overrightarrow{SWB}^{(j)} = <SWB_1^{(j)}, SWB_2^{(j)}, SWB_3^{(j)}, ... SWB_p^{(j)}>$$

Quantifying exhibited personal characteristics

The exhibited personal characteristics represent a dynamic measure - a function of a user's observed activities and behaviors. This is called "exhibited personal characteristics" as they are inferred through activities and behaviors that a user exhibits and that correspond to a specific quantified profile of personal characteristics. Present invention categorizes the exhibited personal characteristics as:

$$\overrightarrow{PC}_{exhibited}^{(j)} = <PC_{map,1}^{(j)}, PC_{map,2}^{(j)}, PC_{map,3}^{(j)}, ... PC_{map,m}^{(j)}>$$

$$= f\{\overrightarrow{LDV}^{(j)}\}$$

where $PC_{map,1}^{(j)}, PC_{map,2}^{(j)}, PC_{map,3}^{(j)}, ... PC_{map,m}^{(j)}$ represent $m$ mapped personal characteristics scores obtained as a function of observed activities and behaviors of user $j$ namely $\overrightarrow{LDV}^{(j)}$, and $f$ represents the function that maps the behavior and activity characteristics to personal characteristics.

To calculate the $m$ exhibited personal characteristics, in an embodiment, present invention firstly obtains a user's behavior path distribution matrix (also referred as LDM matrix) by concatenating the $k$-dimension second vector with the $k$-dimension second vectors of other users stored in a database (i.e. all users who have used the proposed invention in the past and have consented for their data to be used and/or users that continue using the invention in the present whose data until a given point of time can be used).

The invention then derives or calculates a correlation matrix C by correlating the $m$-dimension first vector with the $k$-dimension second vector, thus containing correlations between behaviors and personal characteristics. This can be obtained by finding the most significant correlations between frequent behaviors defined with $\overrightarrow{LDV}^{(j)}$ and $\overrightarrow{PC}^{(j)}$ or in one embodiment, using theoretical correlations obtained in psychological literature like in *"Goldberg, L. R. (2009). Personality, Demographics, and Self-Reported Behavioral Acts: The Development of Avocational Interest Scales from Estimates of the Amount of Time Spent in Interest-Related Activities. Then a miracle occurs: Focusing on behavior in social psychological theory and research, 205"*. This is used to calculate a weight (or effect) matrix W, given by:

$$W = LDM \times C$$

where $\times$ is the matrix multiplication operator, *LDM* is a matrix of dimension $N * k$, C is a matrix of dimension $k * m$, $N$ is the number of users stored in said database, and W is a matrix of dimensions $N * m$.

Each row of $W$ corresponds to the weights (or the effect) of activity on exhibited personal characteristics - given by

$\overrightarrow{w}^{(j)} = <w_1^{(j)}, w_2^{(j)}, w_3^{(j)}, ... w_m^{(j)}>$, $\overrightarrow{w}^{(j)}$ represents the change above or below the median personal characteristics, that are exhibited by a user's *LDV*. Thus, the exhibited personal characteristics of each user are obtained as the sum of the median personal characteristics, $\overrightarrow{PC}_{med}$ and the Hadamard product ($\cdot$) of user weights with the median personal characteristics, i.e.:

$$\overrightarrow{PC}_{exhibited}^{(j)} = \overrightarrow{PC}_{med} + \overrightarrow{PC}_{med} \cdot \overrightarrow{w}^{(j)}$$

$$= \overrightarrow{PC}_{med}(1 + \overrightarrow{w}^{(j)})$$

Other embodiments can use a range of different mathematical models for computing $PC_{exhibited}$. Thus, present invention can compute the miss-alignment parameter or 'Delta' $\vec{\Delta}^{(j)}$ between an user's exhibited behaviors (using $\overrightarrow{PC}^{(j)}_{exhibited}$) and their personal characteristics $\overrightarrow{PC}^{(j)}$ as: $\vec{\Delta}^{(j)} = \left| \overrightarrow{PC}^{(j)}_{exhibited} - \overrightarrow{PC}^{(j)} \right|$, where |.| is the absolute value function. The $\vec{\Delta}^{(j)}$ vector represents how miss-aligned an user's behavior is, with respect to each of his/her personal characteristics. Thus, the more the value of a component in $\vec{\Delta}^{(j)}$ is, the more the user behaves away from this component. Since each component of $\vec{\Delta}^{(j)}$ is orthogonal, the cumulative $\Delta^{(j)}$ is given as the Euclidean norm of $\vec{\Delta}^{(j)}$, i.e.,

$$\Delta^{(j)} = \left\| \vec{\Delta}^{(j)} \right\| = \sqrt{\left( \Delta^{(j)}_1 \right)^2 + \left( \Delta^{(j)}_2 \right)^2 + \left( \Delta^{(j)}_3 \right)^2 \dots \left( \Delta^{(j)}_m \right)^2}.$$

Finding a reference group and establishing the recommendation

For each user $x$, present invention creates a group of "role model" users (called the reference group). These users have higher cumulative subjective wellbeing, $SWB^{(j)}$ than user $x$ (i.e. higher third vector) and lower mismatch in their lifestyles, $\Delta^{(j)}$ (i.e. lower miss-alignment parameter) than user $x$. Thus, for user $x$, the reference group $RG^{(x)}$ are given by:

$$RG^{(x)} = \{ \forall j \epsilon \left( SWB^{(x)} < SWB^{(j)} \right) \cap \left( \Delta^{(x)} > \Delta^{(j)} \right) \}$$

In an embodiment, all users that are part of the set $RG^{(x)}$ are now compared against user $x$ taking into account two possible variations of the present invention given by:

1. Similarity in personal characteristics of user $x$ and reference group: In this variation, the personal characteristics of user $x$, $\overrightarrow{PC}^{(x)}$ is compared against the exhibited personal characteristics of each user $j$, $\overrightarrow{PC}^{(j)}$ in the set $RG^{(x)}$ using the cosine similarity measure. This computation determines all the users that have similar static inherent personal characteristics as user $x$, but who have higher $SWB$ and lower $\Delta$. The higher the cosine similarity value is, the higher is the similarity between the two measures being compared. For a user y in $RG^{(x)}$, the cosine similarity $sim(x,y)$ between the users $x$ and $y$ can be defined as:

$$sim(x,y) = \frac{\sum_m (PC^{(x)}_m - \overline{PC}^{(x)})(PC^{(y)}_m - \overline{PC}^{(y)})}{\sqrt{\sum_m (PC^{(x)}_m - \overline{PC}^{(x)})^2 \sum_m (PC^{(y)}_m - \overline{PC}^{(y)})^2}}$$

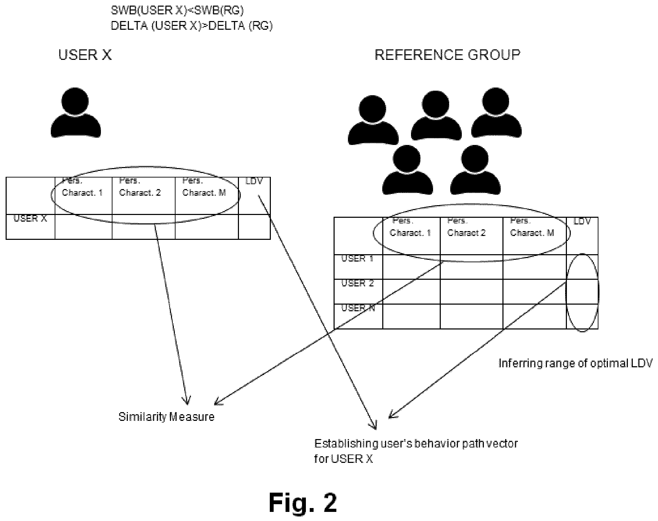

where $PC^{(x)}_m$ is the $m$ personal characteristic of user $x$, $\overline{PC}^{(x)}$ is the average value of all the personal characteristics of user $x$, while $PC^{(y)}_m$ and $\overline{PC}^{(y)}$ represent the same values but for user $y$. This variation of the invention uses the combination of dynamic filtering criteria based on $\Delta$ and calculation of $sim(x, y)$ based on static personal characteristics, and subsequently determines users with the $M$ highest values of $sim(x, y)$ to be deemed the best users to help user $x$ improve his/her activities and behavior. This is represented in Fig. 2.

2. Similarity in personal characteristics of user $x$ and exhibited personal characteristics of reference group: In this variation, the personal characteristics of user $x$, $\overrightarrow{PC}^{(x)}$ is compared against the exhibited personal characteristics of each user $j$, $\overrightarrow{PC}^{(j)}_{exhibited}$ in the set $RG^{(x)}$ using the cosine similarity measure. This computation determines all the users having dynamic behaviors that are well in line with the static personal characteristics as user $x$, but who have higher $SWB$ and lower $\Delta$. As defined above, the higher the cosine similarity value is, the higher is the similarity between the two measures being compared. For a user $y$ in $RG^{(x)}$, the cosine similarity $sim(x, y)$ between the users $x$ and $y$ is defined as:

$$sim(x,y) = \frac{\sum_m (PC^{(x)}_m - \overline{PC}^{(x)})(PC^{(y)}_{exhibited,m} - \overline{PC}^{(y)}_{exhibited})}{\sqrt{\sum_m (PC^{(x)}_m - \overline{PC}^{(x)})^2 \sum_m (PC^{(y)}_{exhibited,m} - PC^{(y)}_{exhibited})^2}},$$

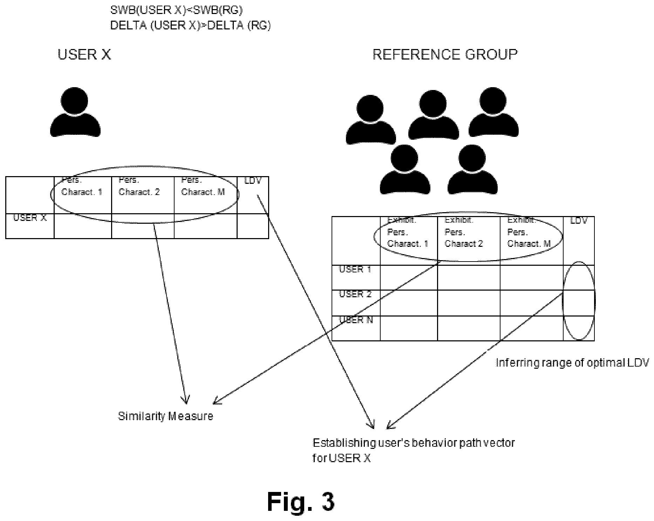

where $PC^{(x)}_m$ and $\overline{PC}^{(x)}$ represent the same values as above, while $PC^{(y)}_{exhibited,m}$ represents the $m$ component of the exhibited personal characteristic and $\overline{PC}^{(y)}_{exhibited}$ represent the average value of the exhibited personal characteristic for user $y$. This variation of the invention uses the combination of dynamic filtering criteria based on $\Delta$ and calculation of $sim(x, y)$ based on exhibited personal characteristics (based on dynamic activities and behaviors), and subsequently determines users with the $M$ highest values of $sim(x, y)$ to be deemed the best users to help user $x$ improve his/her activities and behavior. This is represented in Fig. 3.

Once the top $M$ similar users are identified from either of the above two variations of the present invention, the user's behavior path vector is determined/computed using $LDVs$ of the most similar users. Thus, the range of optimal $LDV$ can be suggested to the user $x$. This recommendation will increase the user $x$'s subjective wellbeing score, while decreasing his/her miss-alignment score.

As indicated above, in one particular embodiment, present invention uses the personality measure using the BIG-5 personality inventory as the personal characteristics. This has 5 components - Extroversion, Agreeableness, Conscientiousness, (Emotional) Stability and Intellect. Thus, for each user: $\vec{PC}^{(j)} = \vec{p}^{(j)} = < e^{(j)}, a^{(j)}, c^{(j)}, s^{(j)}, i^{(j)} >$.

Through the computational process described above for obtaining the exhibited personal characteristics from a person's exhibited lifestyle, the exhibited personality for each user is obtained as:

$$\vec{PC}^{(j)}_{exhibited} = \vec{p}^{(j)}_{exhibited} = < e^{(j)}_{map}, a^{(j)}_{map}, c^{(j)}_{map}, s^{(j)}_{map}, i^{(j)}_{map} >$$

Thus, the miss-alignment between one's lifestyle and personality is:

$$\Delta^{(j)} = \left\| \vec{\Delta}^{(j)} \right\| = \left\| \vec{p}^{(j)}_{exhibited} - \overline{p}^{(j)} \right\|$$

$$= \sqrt{\left(e^{(j)} - e^{(j)}_{map}\right)^2 + \left(a^{(j)} - a^{(j)}_{map}\right)^2 + \left(c^{(j)} - c^{(j)}_{map}\right)^2 + \left(s^{(j)} - s^{(j)}_{map}\right)^2 + \left(i^{(j)} - i^{(j)}_{map}\right)^2}$$

The reference group determination and similarity measure calculation for the recommendation system are performed as described above, with $1 \le m \le 5$.

The present invention has been described in particular detail with respect to specific possible embodiments. Those of skill in the art will appreciate that the invention may be practiced in other embodiments. For example, the nomenclature used for components, capitalization of component designations and terms, the attributes, data structures, or any other programming or structural aspect is not significant, mandatory, or limiting, and the mechanisms that implement the invention or its features can have various different names, formats, and/or protocols. Further, the system and/or functionality of the invention may be implemented via various combinations of software and hardware, as described, or entirely in software elements. Also, particular divisions of functionality between the various components described herein are merely exemplary, and not mandatory or significant. Consequently, functions performed by a single component may, in other embodiments, be performed by multiple components, and functions performed by multiple components may, in other embodiments, be performed by a single component.

Certain aspects of the present invention include process steps or operations and instructions described herein in an algorithmic and/or algorithmic-like form. It should be noted that the process steps and/or operations and instructions of the present invention can be embodied in software, firmware, and/or hardware, and when embodied in software, can be downloaded to reside on and be operated from different platforms used by real-time network operating systems.

In the discussion above, certain aspects of one embodiment include process steps and/or operations and/or instructions described herein for illustrative purposes in a particular order, in particular the reception or capture of the first, second and third data. However, the particular order shown and discussed herein is illustrative only and not limiting. Those of skill in the art will recognize that other orders of the process steps are possible and, in some embodiments, one or more of the process steps discussed above can be combined. Consequently, the particular order discussed herein does not limit the scope of the invention as claimed below.

**Claims**

1.   A computer implemented method for dynamically modelling personal characteristics of a user based on observed behaviors and activities of the user, the method comprising:

   a) receiving, by a computing system, first data regarding different personal characteristics of a user, providing a first vector of personal characteristics of said user based on said received first data;

b) receiving, by the computing system, second data regarding behavior and activity characteristics of the user, providing a second vector of behavior and activity characteristics of the user based on said received second data;

c) receiving, by the computing system, third data regarding one or more subjective wellbeing measures of the user, providing a third vector of wellbeing measures of the user based on said received third data;

d) calculating, by the computing system, exhibited personal characteristics of the user using the first and second vectors of the user by means of:

- calculating a behavior and activity distribution matrix LDM of the user by concatenating the second vector of the user with second vectors of other users stored in a database;
- calculating a correlation matrix C by correlating the first vector with the second vector;
- calculating a weight matrix by implementing the following equation: W = LDM $\times$ C , where $\times$ is the matrix multiplication operator; and
- calculating the exhibited personal characteristics by implementing the following equation:

$$\overrightarrow{PC}_{exhibited}^{(j)} = \overrightarrow{PC}_{med} + \overrightarrow{PC}_{med} \cdot \overrightarrow{w}^{(j)} = \overrightarrow{PC}_{med}(1 + \overrightarrow{w}^{(j)}),$$ where $\overrightarrow{PC}_{med}$ is the median of the first vector and $(\cdot)$ is the Hadamard product of the weights of the user with said median of the first vector;

e) calculating, by the computing system, a miss-alignment parameter between the calculated exhibited personal characteristics and the first vector of the user;

f) creating, by the computing system, a reference group for the user, said reference group comprising a plurality of users having a higher third vector and lower miss-alignment parameter than the user;

g) implementing, by the computing system, a similarity measure between the user and the reference group to identify which of said plurality of users within the reference group has more characteristics in common with the user;

h) determining, by the computing system, from the most similar users of said reference group, an optimal behavior and activity distribution vector for the user, and using the optimal behavior and activity distribution vector to recommend behavior and activity modifications to the user to improve the user's wellbeing.

2. The method of claim 1, wherein the similarity measure of step g) being calculated by comparing the first vector of the user with a first vector of each one of the plurality of users within the reference group using a cosine similarity measure, the first vector of each one of the plurality of users being calculated using personal characteristics of each user.

3. The method of claim 1, wherein the similarity measure of step g) being calculated by comparing the first vector of the user with calculated exhibited personal characteristics of each one of the plurality of users within the reference group using a cosine similarity measure, the exhibited personal characteristics of each one of the plurality of users being calculated using personal characteristics and behavior and activity characteristics of each user.

4. The method of claim 1, wherein the first data being received from a server providing results of a personality survey implemented by the user.

5. The method of claim 4, wherein the first data being further inferred through knowledge of variables of the user including people from a same neighborhood or community and/or by using a clustering or machine learning algorithm on said variables.

6. The method of claim 1, wherein the second data being received as a vector quantifying a proportion of the different daily activities of the user where each activity is assigned with a specific value or with a specific percentage.

7. The method of claim 6, wherein the second data being received from a server providing a self-report of the user and/or from a passive sensor, mobile phone, wearable device or activity recognition system of the user.

8. The method of claim 1, wherein the third data being received from a sensor monitoring activities or physiological signals of the user including body temperature, heart rate parameters, voice parameters and/or facial expressions.

9. A system for dynamically modelling personal characteristics of a user based on observed behaviors and activities of the user, comprising:

a memory; and

one or more processors,

wherein the one or more processors being arranged and configured to:

receive first data regarding different personal characteristics of a user, providing a first vector of personal characteristics of said user based on said received first data;
receive second data regarding behavior and activity characteristics of the user, providing a second vector of behavior and activity characteristics of the user based on said received second data;
receive third data regarding one or more subjective wellbeing measures of the user, providing a third vector of wellbeing measures of the user based on said received third data;
calculate exhibited personal characteristics of the user using the first and second vectors of the user by means of:

- calculating a behavior and activity distribution matrix LDM of the user by concatenating the second vector of the user with second vectors of other users stored in a database;
- calculating a correlation matrix C by correlating the first vector with the second vector;
- calculating a weight matrix by implementing the following equation: W = LDM $\times$ C , where $\times$ is the matrix multiplication operator; and
- calculating the exhibited personal characteristics by implementing the following equation:

$$\overrightarrow{PC}_{exhibited}^{(j)} = \overrightarrow{PC}_{med} + \overrightarrow{PC}_{med} \cdot \vec{w}^{(j)} = \overrightarrow{PC}_{med}(1 + \vec{w}^{(j)}),$$ where $\overrightarrow{PC}_{med}$ is the median of the first vector and $(\cdot)$ is the Hadamard product of the weights of the user with said median of the first vector;

calculate a miss-alignment parameter between the calculated exhibited personal characteristics and the first vector of the user;
create a reference group for the user, said reference group comprising a plurality of users having a higher third vector and lower miss-alignment parameter than the user;
implement a similarity measure between the user and the reference group to identify which of said plurality of users within the reference group has more characteristics in common with the user;
determine, from the most similar users of said reference group, an optimal behavior and activity distribution vector for the user, and using the optimal behavior and activity distribution vector to recommend behavior and activity modifications to the user to improve the user's wellbeing.

10. The system of claim 9, further comprising a server configured to provide the second data to the one or more processors in the form of a self-report of the user and/or a passive sensor, mobile phone, wearable device or activity recognition system of the user to provide the second data to the one or more processors.

11. The system of claim 9 or 10, further comprising a sensor configured to monitor activities or physiological signals of the user including body temperature, heart rate parameters, voice parameters and/or facial expressions, the third data being received from said sensor.

12. The system of claims 9 to 11, further comprising a server configured to provide the first data to the one or more processors in the form of a personality survey implemented by the user.

13. A computer program product comprising non-transitory computer readable medium that when executed by one or more processors of a computer system implement the method of claims 1 to 8.

**Patentansprüche**

1. Computerimplementiertes Verfahren zum dynamischen Modellieren persönlicher Charakteristika eines Benutzers basierend auf beobachteten Verhaltensweisen und Aktivitäten des Benutzers, wobei das Verfahren umfasst:

a) Empfangen, durch ein Datenverarbeitungssystem, von ersten Daten bezüglich unterschiedlicher persönlicher Charakteristika, Bereitstellen eines ersten Vektors von persönlichen Charakteristika des Benutzers basierend auf den empfangenen ersten Daten;
b) Empfangen, durch das Datenverarbeitungssystem, von zweiten Daten bezüglich Verhaltens- und Aktivitätscharakteristika des Benutzers, Bereitstellen eines zweiten Vektors von Verhaltens- und Aktivitätscharakteristika des Benutzers basierend auf den empfangenen zweiten Daten;

c) Empfangen, durch das Datenverarbeitungssystem, von dritten Daten bezüglich eines oder mehrerer subjektiver Wohlfühlmaße des Benutzers, Bereitstellen eines dritten Vektors von Wohlfühlmaßen des Benutzers basierend auf den empfangenen dritten Daten;

d) Berechnen, durch das Datenverarbeitungssystem, von gezeigten persönlichen Charakteristika des Benutzers unter Verwendung des ersten und zweiten Vektors des Benutzers mittels:

- Berechnen einer Verhaltens- und Aktivitätsverteilungsmatrix LDM des Benutzers durch Verketten des zweiten Vektors des Benutzers mit zweiten Vektoren anderer Benutzer, die in einer Datenbank gespeichert sind;
- Berechnen einer Korrelationsmatrix C durch Korrelieren des ersten Vektors mit dem zweiten Vektor;
- Berechnen einer Gewichtungsmatrix durch Implementieren der folgenden Gleichung: $W = LDM \times C$, wobei x der Multiplikationsoperator der Matrix ist; und
- Berechnen der gezeigten persönlichen Charakteristika durch Implementieren der folgenden Gleichung:

$$\overrightarrow{PC}^{(j)}_{gezeigt} = \overrightarrow{PC}_{med} + \overrightarrow{PC}_{med} \cdot \vec{w}^{(j)} = \overrightarrow{PC}_{med}(1 + \vec{e}^{(j)}),$$ **wobei** $\overrightarrow{PC}_{med}$ der Median des ersten Vektors ist und $(\cdot)$ das Hadamard-Produkt der Gewichtungen des Benutzers mit dem Median des ersten Vektors ist;

e) Berechnen, durch das Datenverarbeitungssystem, eines Fehlausrichtungsparameters zwischen den berechneten gezeigten persönlichen Charakteristika und dem ersten Vektor des Benutzers;

f) Erstellen, durch das Datenverarbeitungssystem, einer Referenzgruppe für den Benutzer, wobei die Referenzgruppe eine Vielzahl von Benutzern umfasst, die einen höheren dritten Vektor und einen niedrigeren Fehlausrichtungsparameter als der Benutzer aufweisen;

g) Implementieren, durch das Datenverarbeitungssystem, eines Ähnlichkeitsmaßes zwischen dem Benutzer und der Referenzgruppe, um zu erkennen, welcher der Vielzahl von Benutzern innerhalb der Referenzgruppe mehr gemeinsame Charakteristika mit dem Benutzer aufweist;

h) Bestimmen, durch das Datenverarbeitungssystem, eines optimalen Verhaltens- und Aktivitätsverteilungsvektors für den Benutzer aus den ähnlichsten Benutzern der Referenzgruppe und Verwenden des optimalen Verhaltens- und Aktivitätsverteilungsvektors, um dem Benutzer Verhaltens- und Aktivitätsänderungen zu empfehlen, um das Wohlbefinden des Benutzers zu verbessern.

2. Verfahren nach Anspruch 1, wobei das Ähnlichkeitsmaß von Schritt g) durch Vergleichen des ersten Vektors des Benutzers mit einem ersten Vektor eines jeden der Vielzahl von Benutzern innerhalb der Referenzgruppe unter Verwendung eines Kosinus-Ähnlichkeitsmaßes berechnet wird, wobei der erste Vektor eines jeden der Vielzahl von Benutzern unter Verwendung persönlicher Charakteristika jedes Benutzers berechnet wird.

3. Verfahren nach Anspruch 1, wobei das Ähnlichkeitsmaß von Schritt g) durch Vergleichen des ersten Vektors des Benutzers mit berechneten gezeigten persönlichen Charakteristika eines jeden der Vielzahl von Benutzern innerhalb der Referenzgruppe unter Verwendung eines Kosinus-Ähnlichkeitsmaßes berechnet wird, wobei die gezeigten persönlichen Charakteristika eines jeden der Vielzahl von Benutzern unter Verwendung persönlicher Charakteristika und Verhaltens- und Aktivitätscharakteristika jedes Benutzers berechnet werden.

4. Verfahren nach Anspruch 1, wobei die ersten Daten von einem Server empfangen werden, der Ergebnisse einer durch den Benutzer implementierten Persönlichkeitsumfrage bereitstellt.

5. Verfahren nach Anspruch 4, wobei die ersten Daten ferner durch Kenntnis von Variablen des Benutzers einschließlich Menschen aus derselben Nachbarschaft oder Gemeinschaft und/oder durch Verwendung eines Clustering-Algorithmus oder Maschinenlernalgorithmus an den Variablen abgeleitet werden.

6. Verfahren nach Anspruch 1, wobei die zweiten Daten als Vektor empfangen werden, der einen Anteil der unterschiedlichen täglichen Aktivitäten des Benutzers quantifiziert, wobei jeder Aktivität ein bestimmter Wert oder ein bestimmter Prozentsatz zugeordnet ist.

7. Verfahren nach Anspruch 6, wobei die zweiten Daten von einem Server, der eine Selbsteinschätzung des Benutzers bereitstellt, und/oder von einem passiven Sensor, einem Mobiltelefon, einer am Körper tragbaren Vorrichtung oder einem Aktivitätserkennungssystem des Benutzers empfangen werden.

8. Verfahren nach Anspruch 1, wobei die dritten Daten von einem Sensor empfangen werden, der Aktivitäten oder physiologische Signale des Benutzers überwacht einschließlich Körpertemperatur, Herzfrequenzparametern, Sprachparametern und/oder Gesichtsausdrücken überwacht.

9. System zum dynamischen Modellieren persönlicher Charakteristika eines Benutzers basierend auf beobachteten Verhaltensweisen und Aktivitäten des Benutzers, das umfasst:

einen Speicher; und
einen oder mehrere Prozessoren,
wobei der eine oder die mehreren Prozessoren angeordnet und konfiguriert sind zum:

Empfangen von ersten Daten bezüglich unterschiedlicher persönlicher Charakteristika, Bereitstellen eines ersten Vektors von persönlichen Charakteristika des Benutzers basierend auf den empfangenen ersten Daten;
Empfangen von zweiten Daten bezüglich Verhaltens- und Aktivitätscharakteristika des Benutzers, Bereitstellen eines zweiten Vektors von Verhaltens- und Aktivitätscharakteristika des Benutzers basierend auf den empfangenen zweiten Daten;
Empfangen von dritten Daten bezüglich eines oder mehrerer subjektiver Wohlfühlmaße des Benutzers, Bereitstellen eines dritten Vektors von Wohlfühlmaßen des Benutzers basierend auf den empfangenen dritten Daten;
Berechnen von gezeigten persönlichen Charakteristika des Benutzers unter Verwendung des ersten und zweiten Vektors des Benutzers mittels:

- Berechnen einer Verhaltens- und Aktivitätsverteilungsmatrix LDM des Benutzers durch Verketten des zweiten Vektors des Benutzers mit zweiten Vektoren anderer Benutzer, die in einer Datenbank gespeichert sind;
- Berechnen einer Korrelationsmatrix C durch Korrelieren des ersten Vektors mit dem zweiten Vektor;
- Berechnen einer Gewichtungsmatrix durch Implementieren der folgenden Gleichung: W = LDM x C, wobei x der Multiplikationsoperator der Matrix ist; und
- Berechnen der gezeigten persönlichen Charakteristika durch Implementieren der folgenden Gleichung: $\overrightarrow{PC}^{(j)}_{gezeigt} = \overrightarrow{PC}_{med} + \overrightarrow{PC}_{med} \cdot \vec{w}^{(j)} = \overrightarrow{PC}_{med}(1 + \vec{w}^{(j)})$, **wobei** $\overrightarrow{PC}_{med}$ der Median des ersten Vektors ist und $(\cdot)$ das Hadamard-Produkt der Gewichtungen des Benutzers mit dem Median des ersten Vektors ist;

Berechnen eines Fehlausrichtungsparameters zwischen den berechneten gezeigten persönlichen Charakteristika und dem ersten Vektor des Benutzers;
Erstellen einer Referenzgruppe für den Benutzer, wobei die Referenzgruppe eine Vielzahl von Benutzern umfasst, die einen höheren dritten Vektor und einen niedrigeren Fehlausrichtungsparameter als der Benutzer aufweisen;
Implementieren eines Ähnlichkeitsmaßes zwischen dem Benutzer und der Referenzgruppe, um zu erkennen, welcher der Vielzahl von Benutzern innerhalb der Referenzgruppe mehr gemeinsame Charakteristika mit dem Benutzer aufweist;
Bestimmen eines optimalen Verhaltens- und Aktivitätsverteilungsvektors für den Benutzer aus den ähnlichsten Benutzern der Referenzgruppe und Verwenden des optimalen Verhaltens- und Aktivitätsverteilungsvektors, um dem Benutzer Verhaltens- und Aktivitätsänderungen zu empfehlen, um das Wohlbefinden des Benutzers zu verbessern.

10. System nach Anspruch 9, das ferner einen Server umfasst, der konfiguriert ist, um die zweiten Daten dem einen oder den mehreren Prozessoren in Form einer Selbsteinschätzung des Benutzers und/oder eines passiven Sensors, eines Mobiltelefons, einer am Körper tragbaren Vorrichtung oder eines Aktivitätserkennungssystems des Benutzers bereitzustellen.

11. System nach Anspruch 9 oder 10, das ferner einen Sensor umfasst, der konfiguriert ist, um Aktivitäten oder physiologische Signale des Benutzers einschließlich Körpertemperatur, Herzfrequenzparametern, Stimmenparametern und/oder Gesichtsausdrücken zu überwachen, wobei die dritten Daten von dem Sensor empfangen werden.

12. System nach einem der Ansprüche 9 bis 11, das ferner einen Server umfasst, der konfiguriert ist, um die ersten Daten dem einen oder den mehreren Prozessoren in Form einer durch den Benutzer implementierten Persönlichkeitsumfrage bereitzustellen.

**13.** Computerprogrammprodukt, das ein nichtflüchtiges computerlesbares Medium umfasst, das, wenn es durch einen oder mehrere Prozessoren eines Computersystems ausgeführt wird, das Verfahren der Ansprüche 1 bis 8 implementiert.

**Revendications**

**1.** Procédé mis en œuvre par ordinateur permettant de modéliser dynamiquement des caractéristiques personnelles d'un utilisateur en fonction de comportements et d'activités observés de l'utilisateur, le procédé comprenant :

a) la réception, par un système informatique, de premières données concernant différentes caractéristiques personnelles d'un utilisateur, fournissant un premier vecteur de caractéristiques personnelles dudit utilisateur en fonction desdites premières données reçues ;
b) la réception, par le système informatique, de deuxièmes données concernant des caractéristiques de comportements et d'activités de l'utilisateur, fournissant un deuxième vecteur de caractéristiques de comportements et d'activités de l'utilisateur en fonction des deuxièmes données reçues ;
c) la réception, par le système informatique, de troisièmes données concernant une ou plusieurs mesures subjectives du bien-être de l'utilisateur, fournissant un troisième vecteur de mesures du bien-être de l'utilisateur en fonction desdites troisièmes données reçues ;
d) le calcul, par le système informatique, de caractéristiques personnelles montrées de l'utilisateur à l'aide des premier et deuxième vecteurs de l'utilisateur au moyen de :

- le calcul d'une matrice de distribution LDM de comportements et d'activités de l'utilisateur par concaténation du deuxième vecteur de l'utilisateur avec des deuxièmes vecteurs d'autres utilisateurs stockés dans une base de données ;
- le calcul d'une matrice de corrélation C par corrélation du premier vecteur avec le deuxième vecteur;
- le calcul d'une matrice de pondération par application de l'équation suivante : W = LDM x C, où x est l'opérateur de multiplication de la matrice ; et
- le calcul des caractéristiques personnelles montrées par mise en œuvre de l'équation suivante :

$$\overrightarrow{PC}_{exhibited}^{(j)} = \overrightarrow{PC}_{med} + \overrightarrow{PC}_{med} \cdot \vec{w}^{(j)} = \overrightarrow{PC}_{med}(1 + \vec{w}^{(j)}),$$ où $\overrightarrow{PC}_{med}$ est la médiane du premier vecteur et (·) est le produit de Hadamard des pondérations de l'utilisateur avec ladite médiane du premier vecteur ;

e) le calcul, par le système informatique, d'un paramètre de désalignement entre les caractéristiques personnelles montrées calculées et le premier vecteur de l'utilisateur ;
f) la création, par le système informatique, d'un groupe de référence pour l'utilisateur, ledit groupe de référence comprenant une pluralité d'utilisateurs ayant un troisième vecteur plus grand et un paramètre de désalignement plus petit que l'utilisateur ;
g) la mise en œuvre, par le système informatique, d'une mesure de similarité entre l'utilisateur et le groupe de référence pour identifier lequel de ladite pluralité d'utilisateurs à l'intérieur du groupe de référence a le plus de caractéristiques en commun avec l'utilisateur ;
h) la détermination, par le système informatique, à partir des utilisateurs les plus similaires dudit groupe de référence, d'un vecteur de distribution optimale de comportements et d'activités pour l'utilisateur, et l'utilisation du vecteur de distribution optimale de comportements et d'activités pour recommander des modifications de comportements et d'activités à l'utilisateur pour d'améliorer le bien-être de l'utilisateur.

**2.** Procédé selon la revendication 1, dans lequel la mesure de similarité de l'étape g) étant calculée en comparant le premier vecteur de l'utilisateur avec un premier vecteur de chacun de la pluralité d'utilisateurs à l'intérieur du groupe de référence à l'aide d'une mesure de similarité cosinusoïdale, le premier vecteur de chacun de la pluralité d'utilisateurs étant calculé à l'aide de caractéristiques personnelles de chaque utilisateur.

**3.** Procédé selon la revendication 1, dans lequel la mesure de similarité de l'étape g) étant calculée en comparant le premier vecteur de l'utilisateur avec des caractéristiques personnelles montrées calculées de chacun de la pluralité d'utilisateurs à l'intérieur du groupe de référence à l'aide d'une mesure de similarité cosinusoïdale, les caractéristiques personnelles montrées de chacun de la pluralité d'utilisateurs étant calculées à l'aide de caractéristiques personnelles et de caractéristiques de comportements et d'activités de chaque utilisateur.

**4.** Procédé selon la revendication 1, dans lequel les premières données étant reçues d'un serveur fournissant des résultats d'une enquête de personnalité mise en œuvre par l'utilisateur.

**5.** Procédé selon la revendication 4, dans lequel les premières données étant en outre déduites par le biais de la connaissance de variables de l'utilisateur, y compris des personnes d'un même quartier ou d'une même communauté et/ou par l'utilisation d'un regroupement ou d'un algorithme d'apprentissage automatique sur lesdites variables.

**6.** Procédé selon la revendication 1, dans lequel les deuxièmes données étant reçues en guise de vecteur quantifiant une proportion des différentes activités quotidiennes de l'utilisateur où chaque activité se voit attribuer une valeur spécifique ou un pourcentage spécifique.

**7.** Procédé selon la revendication 6, dans lequel les deuxièmes données étant reçues d'un serveur fournissant un rapport personnel de l'utilisateur et/ou d'un capteur passif, d'un téléphone mobile, d'un dispositif portable ou d'un système de reconnaissance d'activité de l'utilisateur.

**8.** Procédé selon la revendication 1, dans lequel les troisièmes données étant reçues d'un capteur surveillant des activités ou des signaux physiologiques de l'utilisateur, y compris la température corporelle, des paramètres de fréquence cardiaque, des paramètres vocaux et/ou des expressions faciales.

**9.** Système permettant de modéliser dynamiquement des caractéristiques personnelles d'un utilisateur en fonction de comportements et d'activités observés de l'utilisateur, comprenant :

une mémoire ; et
un ou plusieurs processeurs,
dans lequel les un ou plusieurs processeurs étant agencés et configurés pour :

recevoir des premières données concernant différentes caractéristiques personnelles d'un utilisateur, fournissant un premier vecteur de caractéristiques personnelles dudit utilisateur en fonction desdites premières données reçues ;
recevoir des deuxièmes données concernant des caractéristiques de comportements et d'activités de l'utilisateur, fournissant un deuxième vecteur de caractéristiques de comportements et d'activités de l'utilisateur en fonction des deuxièmes données reçues ;
recevoir des troisièmes données concernant une ou plusieurs mesures subjectives du bien-être de l'utilisateur, fournissant un troisième vecteur de mesures du bien-être de l'utilisateur en fonction desdites troisièmes données reçues ;
calculer des caractéristiques personnelles montrées de l'utilisateur à l'aide des premier et deuxième vecteurs de l'utilisateur au moyen de :

- le calcul d'une matrice de distribution LDM de comportements et d'activités de l'utilisateur par concaténation du deuxième vecteur de l'utilisateur avec des deuxièmes vecteurs d'autres utilisateurs stockés dans une base de données ;
- le calcul d'une matrice de corrélation C par corrélation du premier vecteur avec le deuxième vecteur;
- le calcul d'une matrice de pondération par application de l'équation suivante : W = LDM x C, où x est l'opérateur de multiplication de la matrice ; et
- le calcul des caractéristiques personnelles montrées par mise en œuvre de l'équation suivante :

$$\overrightarrow{PC}_{exhibited}^{(j)} = \overrightarrow{PC}_{med} + \overrightarrow{PC}_{med} \cdot \vec{w}^{(j)} = \overrightarrow{PC}_{med}(1 + \vec{w}^{(j)})$$, où $\overrightarrow{PC}_{med}$ est la médiane du premier vecteur et $(\cdot)$ est le produit de Hadamard des pondérations de l'utilisateur avec ladite médiane du premier vecteur ;

calculer un paramètre de désalignement entre les caractéristiques personnelles montrées calculées et le premier vecteur de l'utilisateur ;
créer un groupe de référence pour l'utilisateur, ledit groupe de référence comprenant une pluralité d'utilisateurs ayant un troisième vecteur plus grand et un paramètre de désalignement plus petit que l'utilisateur ;
mettre en œuvre une mesure de similarité entre l'utilisateur et le groupe de référence pour identifier lequel de ladite pluralité d'utilisateurs à l'intérieur du groupe de référence a le plus de caractéristiques en commun avec l'utilisateur ;
déterminer, à partir des utilisateurs les plus similaires dudit groupe de référence, d'un vecteur de distribution optimale de comportements et d'activités pour l'utilisateur, et l'utilisation du vecteur de distribution optimale de comportements et d'activités pour recommander des modifications de comportements et d'activités à l'utilisateur pour d'améliorer le bien-être de l'utilisateur.

10. Système selon la revendication 9, comprenant en outre un serveur configuré pour fournir les deuxièmes données aux un ou plusieurs processeurs sous la forme d'un rapport personnel de l'utilisateur et/ou d'un capteur passif, d'un téléphone mobile, d'un dispositif portable ou d'un système de reconnaissance d'activité de l'utilisateur pour fournir les deuxièmes données aux un ou plusieurs processeurs.

11. Système selon la revendication 9 ou 10, comprenant en outre un capteur configuré pour surveiller des activités ou des signaux physiologiques de l'utilisateur, y compris la température corporelle, des paramètres de fréquence cardiaque, des paramètres vocaux et/ou des expressions faciales, les troisièmes données étant reçues dudit capteur.

12. Système selon les revendications 9 à 11, comprenant en outre un serveur configuré pour fournir les premières données aux un ou plusieurs processeurs sous la forme d'une enquête de personnalité mise en œuvre par l'utilisateur.

13. Produit programme d'ordinateur comprenant un support non transitoire lisible par ordinateur qui lorsqu'il est exécuté par un ou plusieurs processeurs d'un système d'ordinateur met en œuvre le procédé selon les revendications 1 à 8.

Fig. 1

SWB(USER X)<SWB(RG)
DELTA (USER X)>DELTA (RG)

USER X

REFERENCE GROUP

Similarity Measure

Inferring range of optimal LDV

Establishing user's behavior path vector
for USER X

# Fig. 2

Fig. 3

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20170132395 A1
- US 2012059785 A1
- US 7457768 B2
- CN 103309976 B
- US 2017049663 A1
- US 2014335490 A1
- US 2007233631 A1
- CN 107702706 A